# FASCICULE DE BREVET EUROPEEN

(11) **EP 2 124 736 B1**
(45) Date de publication et mention de la délivrance du brevet: **31.10.2012**
(21) Numéro de dépôt: 08717066.8
(22) Date de dépôt: 22.02.2008
(51) Int. Cl.: A61B 5/05, A61B 5/053

(54) **SYSTEME D'ANALYSE ELECTROPHYSIOLOGIQUE**
ELEKTROPHYSIOLOGISCHES ANALYSENSYSTEM
ELECTROPHYSIOLOGICAL ANALYSIS SYSTEM

(30) Priorité: 23.02.2007 FR 0753461
(43) Date de publication de la demande: 02.12.2009
(73) Titulaire: Impeto Medical, 75014 Paris (FR)
(72) Inventeur: BRUNSWICK, Philippe, 75014 Paris (FR); BOCQUET, Nicolas, 92320 Chatillon (FR)
(74) Mandataire: Le Forestier, Eric
(86) Numéro de dépôt international: PCT/EP2008/052211
(87) Numéro de publication internationale: WO 2008/107324

(56) Documents cités:
- WO-A-2006/136598
- US-A- 4 690 152
- US-A1- 2006 085 049

## Description

La présente invention concerne d'une façon générale les systèmes et procédés d'utilisation des systèmes dans le domaine de la santé humaine ou animale.

Compte tenu du coût des analyses de sang et du caractère invasif des prélèvements sanguins préalables, les médecins sont aujourd'hui de plus en plus réticents à prescrire à leurs patients des bilans complets trop fréquents.

Il en résulte une évidente sous-détection de certaines maladies dont la détection s'effectue principalement par analyse de sang, telles que le diabète, l'hypertension, l'hyperthyroïdie, les maladies des coronaires, ....

De plus lorsque ces maladies sont détectées, il est souvent très difficile ou coûteux d'évaluer l'efficacité du traitement prescrit, car il est impossible dans la pratique d'effectuer des analyses biologiques journalières.

On connaît par ailleurs différents systèmes de mesure électrophysiologique tels que les appareils d'électrocardiographie et d'électroencéphalographie. Ces systèmes sont passifs en ce sens qu'ils mesurent des phénomènes électriques générés naturellement par le corps humain, qui ont l'avantage d'être non invasifs mais dont les possibilités en termes de diagnostic sont limitées.

On connaît également des systèmes de mesure électrophysiologique dits actifs, à base d'impédancemétrie. Le principe de fonctionnement de ces appareils consiste à faire circuler des courants entre différentes électrodes placées sur le corps, et à examiner la manière dont certaines régions du corps atténuent ce courant. En effet ces techniques, à fréquences assez élevées, ont l'inconvénient de dépendre fortement de l'interface peau électrode et en particulier de son effet capacitif. La reproductibilité des mesures entre patients voire sur un même patient est sujette à caution pour ces mesures à haute fréquence. Les mesures en très basses fréquences, elles, peuvent être nocives pour les cellules.

Par ailleurs on connaît déjà des systèmes de diagnostic dans lesquels on applique à des électrodes placées sur les doigts d'une même main des créneaux de tension rectangulaire d'une certaine fréquence, nécessairement élevée pour pouvoir détecter les phénomènes capacitifs au niveau de la peau, et l'on étudie le courant qui circule dans cette partie du corps en réponse à cette tension rectangulaire. Ce système a évolué pour intégrer, en liaison avec la fréquence élevée des créneaux, une analyse de Fourier qui donne la répartition spectrale du courant relevé. Toutefois, ce système connu a des applications limitées à des détections très localisées (au voisinage des doigts des mains et des pieds), et ne permet d'effectuer des diagnostics que dans les limites des techniques conventionnelles d'acupuncture.

La Demanderesse est déjà parvenue à élargir les possibilités de diagnostic des systèmes de type électrophysiologique, en leur permettant de détecter un certain nombre de maladies, pathologies, terrains pathologiques ou autres troubles détectables habituellement par analyse de sang ou autre fluide corporel.

Elle a ainsi développé un système de diagnostic non invasif, simple d'emploi ayant une spécificité et une sensibilité équivalentes aux analyses de laboratoires et qui permet de détecter avec une fiabilité améliorée et avec un éventail de possibilités plus large certaines pathologies, certaines prédispositions pathologiques ou certains dysfonctionnements d'organes.

Ce système d'analyse est décrit dans le document FR-A-2 887 427.

La présente invention vise à proposer un système amélioré, qui en particulier permette de prendre en compte l'évolution des phénomènes électrochimiques se produisant dans le corps en fonction du niveau de tension auquel les électrodes sont assujetties.

L'invention vise également à proposer un procédé de traitement de données en vue de l'élaboration d'un diagnostic, qui soit capable de prendre en compte des mesures révélant de tels phénomènes électrochimiques.

On propose à cet effet selon un premier aspect un système d'analyse électrophysiologique notamment pour la détection d'états pathologiques, comprenant:
une série d'électrodes aptes à être placées en différentes régions éloignées du corps humain,
une source de tension continue ajustable et apte à engendrer en réponse au circuit de commande des créneaux successifs d'une tension continue qui varie d'un créneau à l'autre, la durée des créneaux étant supérieure ou égale à environ 0,2 seconde,
un circuit de commutation apte à sélectivement relier une paire d'électrodes dites actives à la source de tension, et à connecter au moins une autre électrode en haute impédance, et
un circuit de mesure apte à relever des données représentatives du courant dans les électrodes actives et des potentiels sur au moins certaines électrodes connectées en haute impédance en réponse à l'application desdits créneaux,
la gamme des tensions couvertes par les créneaux étant propre à provoquer d'un créneau à l'autre l'apparition ou la disparition de phénomènes électrochimiques au voisinage des électrodes actives.
   * le système comprenant en outre un dispositif de traitement apte à analyser l'évolution réciproque dudit courant et desdits potentiels en fonction de la tension des créneaux, et à comparer une telle évolution avec au moins une évolution de référence.

Certains aspects préférés mais non limitatifs de ce système sont les suivants :
* le circuit de commutation est apte à successivement relier différentes paires d'électrodes actives à ladite source de tension.
* le circuit de commutation est apte, lorsqu'une paire d'électrodes est reliée à la source de tension, à connecter toutes les autres électrodes en haute impédance.
* le circuit de mesure comprend une résistance apte à être connectée entre l'une des électrodes d'une paire active et une tension de référence.
* le système comprend également un circuit de calibration apte, pour une paire d'électrodes actives données, à ajuster la valeur de la résistance de mesure pour qu'elle soit du même ordre de grandeur que la résistance présente entre les deux électrodes actives en présence d'une tension continue.
* le circuit de calibration est apte à ajuster la valeur de la résistance de mesure pour qu'elle soit voisine de la résistance du corps humain.
* les données représentatives du courant dans les électrodes actives dérivent de la différence de potentiel mesurée aux bornes de la résistance de mesure.
* le circuit de mesure est apte à mesurer les potentiels sur toutes les électrodes.
* les créneaux de tension ont une valeur de tension comprise entre environ 1 et 4 volts et une durée comprise entre environ 0,5 et 5 secondes.
* la tension des créneaux successifs varie dans un sens puis dans un autre.
* la tension des créneaux successifs varie avec un premier pas puis avec un deuxième pas plus petit que le premier.
* la tension des créneaux successifs varie par pas compris entre environ 0,05 et 1 volt.
* les créneaux successifs sont espacés d'une durée comprise entre environ 0,5 et 5 secondes.
* le circuit de commutation est apte à relier une même paire d'électrodes à la source de tension selon deux polarités inversées.
* le système comprend deux électrodes pour lobes frontaux gauche et droit, deux électrodes pour mains gauche et droite et deux électrodes pour pieds gauche et droit.
* le circuit de commutation est apte à relier à la source de tension des paires d'électrodes constituées par l'électrode de front gauche et l'électrode de front droit, l'électrode de front droit et l'électrode de front gauche, l'électrode de main gauche et l'électrode de main droite, l'électrode de main droite et l'électrode de main gauche, l'électrode de pied gauche et l'électrode de pied droit, et l'électrode de pied droit et l'électrode de pied gauche.
* après avoir relié une certaine paire d'électrodes à la source de tension avec une certaine polarité, le circuit de commutation est apte à relier cette même paire d'électrodes à la source de tension avec une polarité inversée seulement après qu'une autre paire d'électrodes, distante sur le corps, a été reliée à la source de tension.

On propose selon un deuxième aspect de l'invention un procédé d'utilisation du système selon l'une des revendications 1 à 9 comprenant les étapes suivantes :
- recevoir un jeu de données comprenant des mesures révélatrices de phénomènes électrochimiques au voisinage d'électrodes appliquées sur la peau du patient en des endroits du corps prédéterminés,
- accéder à au moins un jeu de données de référence mémorisé comprenant des mesures révélatrices de phénomènes électrochimiques obtenues dans les mêmes conditions sur des patients identifiés comme atteints ou non atteints de cette pathologie, et
- confronter ledit jeu de donnés reçu avec les jeux de données de référence.

* les données mesurées étant obtenues à partir de valeurs de courant dans des électrodes actives et de valeurs de potentiels sur des électrodes à haute impédance en réponse à l'application de créneaux de tension entre électrodes actives dont le niveau de tension varie d'un créneau à l'autre pour provoquer l'apparition ou la disparition de phénomènes électrochimiques au voisinage des électrodes actives.

Des aspects préférés mais non limitatifs de ce procédé sont les suivants :
* les jeux de données comprennent en outre des données d'ordre physiologique et/ou comportemental et/ou environnemental.
* les jeux de données comprennent des mesures réalisées sur un patient après un effort prédéterminé du patient.
* lesdites mesures sont fournies par un système tel que défini plus haut.
* le procédé est mis en oeuvre dans un équipement informatique distant du système et relié à lui par un canal de communication de données.

D'autres aspects, buts et avantages de la présente invention apparaîtront mieux à la lecture de la description détaillée suivante de modes de réalisation préférés de celle-ci, donnée à titre d'exemple non limitatif et faite en référence aux dessins annexés, sur lesquels :
La figure 1 est un schéma-bloc illustrant les différents éléments fonctionnels d'un système d'acquisition selon la présente invention,
La figure 2 illustre le schéma électrique équivalent du système de la figure 1, lorsque les électrodes sont reliées à différents endroits du corps humain,
Les figures 3A, 3B et 3C sont des images écran schématisées engendrées par un système de traitement de données associé au système d'acquisition de la figure 1, et
Les figures 4A et 4B sont des tableaux de jeux de données de référence mis en oeuvre dans un procédé de traitement de données en vue d'un diagnostic selon l'invention.

On va maintenant décrire un système de diagnostic de mesures électrophysiologiques selon l'invention. Ce système est fondé sur un principe de fonctionnement actif, en mode de courant continu par opposition en particulier aux systèmes d'impédancemétrie de l'art antérieur dont les inconvénients ont été rappelés plus haut.

A la suite d'un certain nombre d'essais, les Demandeurs ont observé que l'application d'une faible tension continue aux bornes d'une paire d'électrodes éloignées (anode et cathode) donnait des comportements électrochimiques différents à l'anode et à la cathode.

En particulier, on a observé que les ions chlorure Cl- réagissent à l'anode, tandis qu'on assiste à la cathode la réaction d'ions H+. Un objet de l'invention est de pouvoir distinguer le comportement de l'anode d'une part et de la cathode d'autre part.

Sur ce principe, les Demandeurs ont découvert qu'il était possible d'utiliser les électrodes de deux manières, l'une en présence d'un courant et sous une différence de potentiel qui peut varier afin d'observer l'évolution des phénomènes électrochimiques à l'anode et à la cathode, et l'autre en présence d'un courant négligeable (en haute impédance) dans au moins une autre électrode afin d'observer l'évolution des potentiels intermédiaires du corps. Ceci permet d'évaluer les chutes de tensions anodique et cathodique séparément, et donc d'évaluer séparément les teneurs en Cl- et en H+.

Des essais sur patients atteints de certaines pathologies ont à cet égard permis de démontrer que des comportements différents, en matière de résistance statique et de tension électrochimique, pouvaient être révélateurs de telles pathologies. Par exemple, certaines variations de pH à la cathode détectées par des variations de résistance peuvent révéler des troubles du type acidose et alcalose, tandis que certaines variations de la concentration en ions chlorure à l'anode peuvent permettre de diagnostiquer des maladies telles que la mucoviscidose.

Ces mesures de concentrations de chlorures et de réactivité de l'équilibre acido-basique sont susceptibles de tracer des dysfonctionnements homéostatiques, hormonaux, vasculaires, métaboliques, etc. Ces dysfonctionnements peuvent eux-mêmes correspondre à des états physiologiques révélateurs de pathologies ou prédispositions pathologiques variées notamment :
- vasculaires (hypertension et athérome extensif) ;
- hormonales (hypo- et hyperthyroïdie, etc.)
- métaboliques (diabète, etc.),
- chroniques (insuffisance rénale, etc.)
mais aussi révélateurs de l'effet de certains traitements médicamenteux ou autres.

Le système de l'invention tel qu'on va le décrire maintenant en détail vise à exploiter les phénomènes précités ou des phénomènes analogues.

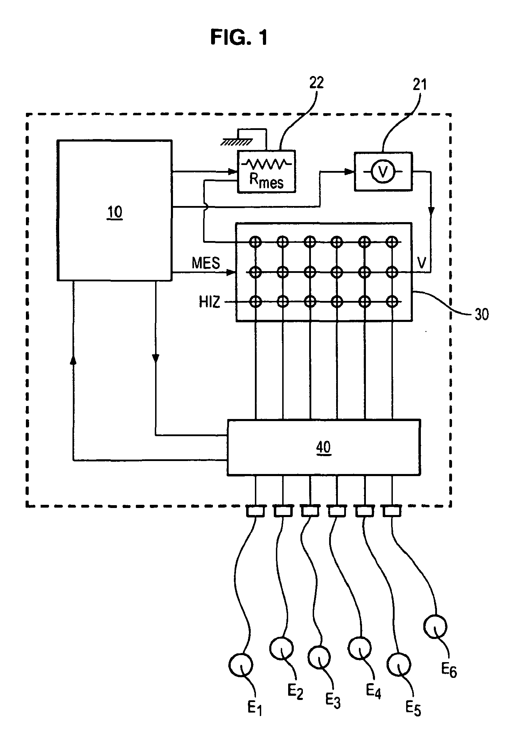

En référence à la figure 1, on a représenté schématiquement un système de diagnostic par mesures électrophysiologiques susceptible d'exploiter les principes précités, qui comprend, dans un boîtier approprié (non illustré), une unité centrale de traitement 10 comprenant par exemple un microprocesseur associé à des mémoires appropriées ainsi qu'à des entrées sorties par exemple pour un clavier de commande et un dispositif d'affichage.

Cette unité centrale commande un générateur de tension électrique 21 capable de produire, en réponse à des signaux de commande appropriés, des créneaux, d'une tension constante mais ajustable pour chacun des créneaux. La durée des créneaux, au minimum de 0,2 seconde et typiquement comprise entre 0,5 et 5 secondes, est éventuellement également ajustable.

L'unité centrale 10 applique également des signaux de commande à un circuit formant résistance de mesure 22 susceptible comme on va le voir d'être reliée entre une électrode et une tension de référence telle que la masse (0 volt), et de présenter à ses bornes une tension à mesurer qui est directement proportionnelle au courant qui la traverse. L'ajustement de la valeur ohmique de cette résistance permet d'optimiser la mesure de tension en fonction du niveau de courant observé, comme on va le voir plus loin.

Ce circuit formant résistance de mesure variable est typiquement un potentiomètre à commande numérique.

Le système comprend également un circuit de commutation électronique susceptible, sur chacune parmi N bornes, soit d'y appliquer les créneaux de tension engendrés par le circuit 21, soit d'y connecter la résistance de mesure 22, soit encore de placer cette borne en haute impédance, où de façon classique en soi elle peut être vue comme étant connectée à la masse par une résistance de valeur ohmique très élevée.

Le circuit de commutation 30 réagit à des signaux de commande fournis par l'unité centrale pour modifier les connexions des N bornes selon différentes séquences, comme on va le voir en détail plus loin.

Le système comprend par ailleurs un circuit de mesure 40 sensible à des instructions de l'unité centrale pour mesurer séquentiellement ou simultanément les tensions présentes à un instant donné sur chacune des N bornes du circuit de commutation. Avantageusement, ce circuit fait appel à des techniques conventionnelles de conversion analogique/numérique sur plusieurs entrées et de multiplexage.

Les N bornes du circuit de commutation sont par ailleurs reliées à N connecteurs permettant la connexion électrique de N électrodes surfaciques En.

Dans le présent exemple de réalisation, le nombre N est égal à 6, et les 6 électrodes connectées sont destinées à être placées dans la région des deux lobes frontaux, des deux mains ou poignets, et des deux pieds ou chevilles d'un patient.

On obtient ainsi un certain nombre de possibilités de mesure, comme on va le voir en détail dans la suite.

L'unité centrale est également apte à effectuer un traitement de l'ensemble des informations et données mises en jeu dans le système, ces données comprenant principalement, en fonction du temps, le niveau de tension engendré au niveau du générateur 21, la valeur de la résistance de mesure 22, et les niveaux de tension relevés, simultanément ou quasi-simultanément, au niveau des six bornes du commutateur et donc au niveau des six électrodes E1 à E6. Ce traitement, comme on va le détailler, a pour objet de détecter et de signaler notamment visuellement l'existence d'un état physiologique lié à une prédisposition pathologique, à la prise ou non d'un traitement, à une pathologie ou autre trouble.

Alternativement, ce traitement de détection peut être effectué dans un équipement déporté, après y avoir chargé (via un support physique ou par communication réseau filaire ou sans fil) l'ensemble des données collectées par l'unité centrale 10 et mémorisées au sein de celle-ci.

Le circuit de commutation électronique 30 permet de sélectionner séquentiellement deux électrodes comme anode Ea et cathode Ec, la première étant reliée au générateur de tension 21 et la seconde étant reliée à la résistance de mesure Rmes de l'unité 22. Pendant ce temps, les autres électrodes (ici les quatre autres, notées Eb1 à Eb4) sont branchées en haute impédance.

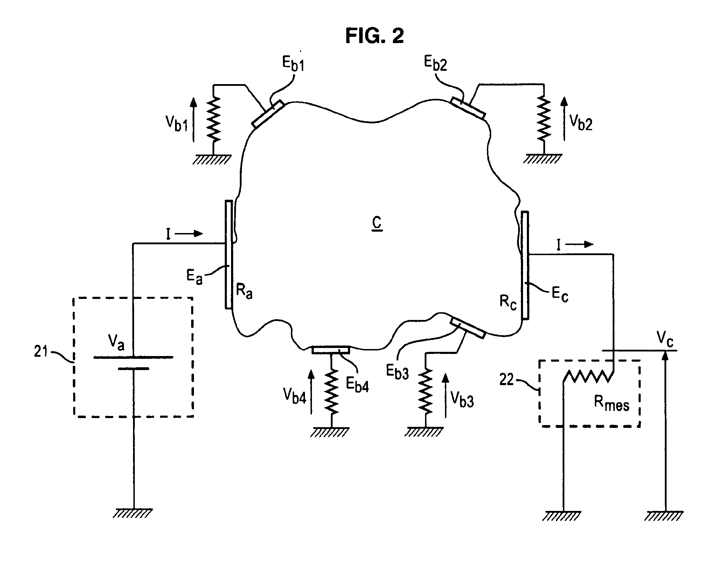

Le circuit électrique équivalent est illustré sur la figure 2. Le circuit de mesure 40 est apte à mesurer, avec une fréquence d'échantillonnage appropriée, par exemple de 100 à 10000 Hz, les tensions présentes sur chacune des 6 électrodes, la tension d'anode Va étant égale à la tension fournie par le générateur de tension 21 tandis que la tension de cathode Vc détermine, par connaissance de la valeur ohmique de la résistance Rmes, la valeur du courant I circulant dans les électrodes Ea et Ec. On néglige ici le courant extrêmement faible qui peut circuler dans les électrodes Eb1 à Eb4, dont le branchement en haute impédance est illustré par les résistances Rb1 à Rb4 d'une valeur typiquement supérieure à 10 MΩ.

Les potentiels sur les électrodes Eb1 à Eb4 sont désignées respectivement par Vb1 à Vb4.

On observera que, dans la pratique, l'unité centrale 10 peut examiner, outre l'évolution dans le temps des tensions Va et Vc, soit l'évolution des quatre tensions Vb1 à Vb4 individuellement, soit l'évolution d'une moyenne de ces valeurs, notée ici Vb.

Le dispositif tel que décrit ci-dessus est commandé par l'unité centrale 10 de façon à effectuer un certain nombre d'opérations.

La première opération est une calibration automatique, effectuée une fois que les électrodes ont été installées sur le patient.

Ainsi, pour chaque paire d'électrodes qui seront utilisées comme couple (anode, cathode), et par exemple la paire d'électrodes de front, la paire d'électrodes de mains et la paire d'électrodes de pieds, le dispositif 10 provoque l'application à l'anode d'une tension continue par exemple de l'ordre de 2 volts, et ajuste la valeur de la résistance Rmes de façon à obtenir une tension de cathode stabilisée d'environ la moitié (ou une fraction déterminée de préférence comprise entre 0,1 et 0,9) de la tension d'anode. Ceci revient à ajuster Rmes pour qu'elle soit voisine (au moins du même ordre de grandeur) de la résistance présente entre ces deux électrodes, qui cumule les résistances de contact électrode/peau et la résistance du corps entre les deux régions distantes du corps. Les valeurs optimales de Rmes pour chacune des paires (anode, cathode) qui seront ensuite utilisées pour les mesures proprement-dites, comme on va le décrire ci-après, sont mémorisées une par une, sous forme de leur signaux de commande, et seront rappelées par l'unité centrale en fonction de la paire (anode, cathode) entrant en jeu.

Cette étape de calibration automatique permet de disposer, pour chaque paire (anode, cathode), de la valeur de Rmes qui offre la meilleure dynamique et la meilleure résolution pour mesurer l'évolution du courant qui passe dans la paire d'électrodes considérée lors des mesures proprement-dites, étant précisé que la résistance présente entre les électrodes peut largement varier en fonction notamment de la surface des électrodes, de la qualité du contact électrode-peau et du vieillissement du matériau des électrodes.

Naturellement, on peut si nécessaire dupliquer ces opérations de calibration automatique pour le cas où une paire d'électrodes anode/cathode devient une paire cathode/anode, ce qui est obtenu en permutant au niveau du circuit de commutation 30 les connexions des deux électrodes.

Les mesures proprement dites s'effectuent en sélectionnant tout d'abord une paire d'électrodes actives (anode, cathode), et en ajustant Rmes sur sa valeur adéquate comme décrit ci-dessus.

Sur l'anode est alors appliquée une série de créneaux de tension continue d'une durée suffisante (de l'ordre de 0,5 à 5 secondes) pour atteindre une certaine stabilisation du courant I, en partant d'un niveau de tension haut (typiquement de l'ordre de 4 volts, dont environ la moitié aux bornes des électrodes pour les raisons expliquées plus haut), jusqu'à un niveau de tension bas (typiquement de l'ordre de 1 à 2 volts), avec un pas de l'ordre de 0,05 à 0,5 volt entre deux créneaux successifs. Une évolution croissante est bien entendu également possible.

Pendant chaque créneau de tension commandé par l'unité centrale 10, celle-ci commande en même temps le circuit de mesure 40 de façon à relever, avec la fréquence d'échantillonnage déterminée (de préférence entre 100 et 10000 Hz comme on l'a indiqué plus haut) les potentiels présents sur chacune des six électrodes.

Parmi ces potentiels, le potentiel Va est représentatif de la tension engendrée par le générateur 21, tandis que le potentiel Vc est directement proportionnel au courant I circulant dans l'anode Ea et la cathode Ec, et permet de le calculer si nécessaire.

Les autres potentiels sont ceux observés aux autres endroits du corps sur lesquels les autres électrodes sont appliquées, mais traversées par un courant essentiellement nul (haute impédance).

Les mesures ci-dessus sont effectuées pour plusieurs paires d'électrodes actives.

Typiquement, avec un système à six électrodes tel que décrit ci-dessus, on effectue les mesures avec les paires d'électrodes suivantes (désignation abrégée entre parenthèses) :

| Anode | Cathode |
|---|---|
| front gauche (FG) | front droit (FD) |
| front droit (FD) | front gauche (FG) |
| main gauche (MG) | main droite (MD) |
| main droite (MD) | main gauche (MG) |
| pied gauche (PG) | pied droit (PD) |
| pied droit (PD) | pied gauche (PG) |

Pour chaque paire d'électrodes, l'évolution du courant pendant chacun des créneaux est liée principalement à la dynamique d'établissement des phénomènes électrochimiques au niveau de l'anode et de la cathode, sous une différence de potentiel à chaque fois différente.

Les niveaux de potentiel et leur évolution dans le temps au cours du créneau non seulement à la cathode (Vc) mais également sur les électrodes passives Eb1 à Eb4 (Vb1 à Vb4) en fonction du niveau de tension Va du créneau considéré, donnent un certain nombre de données brutes qu'il est possible de traiter d'une variété de manières.

Selon une variante avantageuse, on peut prévoir des créneaux de tension dont le niveau de tension varie dans un sens, puis dans l'autre. Plus particulièrement, on peut prévoir que le niveau de tension commence par augmenter avec un pas relativement grossier, par exemple de 0,2 à un volt, puis continue en diminuant, avec un pas réduit par exemple de 0,1 à 0,05 volt une fois que la détection de l'apparition d'un phénomène électrochimique, telle qu'on va la décrire dans la suite, a été effectuée.

A ce jour, un traitement particulièrement utile pour la détection de prédispositions pathologiques, ou pathologies, liées notamment à la concentration en anions chlorure ou à la valeur du pH (concentration en cations hydrogène), consiste à examiner l'évolution de courbes reflétant l'évolution des tensions Vc et Vbi (ou leur moyenne) en fin de créneau, en fonction de la valeur de la tension Va du créneau, et ceci pour chacune des six paires d'électrodes.

Plus précisément, on a observé qu'une telle courbe était initialement pratiquement linéaire, et qu'à partir d'une certaine différence de potentiel entre les électrodes, la linéarité disparaissait, la courbe s'incurvant avec une pente croissante du fait de la naissance de phénomènes électrochimiques.

On a observé également que le niveau de tension d'anode Va à partir duquel la courbe s'incurve, ainsi que le niveau de tension de cathode Vc et les niveaux de tensions d'électrodes inactives Vb1 à Vb4 (ou leur moyenne Vb), étaient eux-mêmes révélateurs de tels ou tels types de troubles.

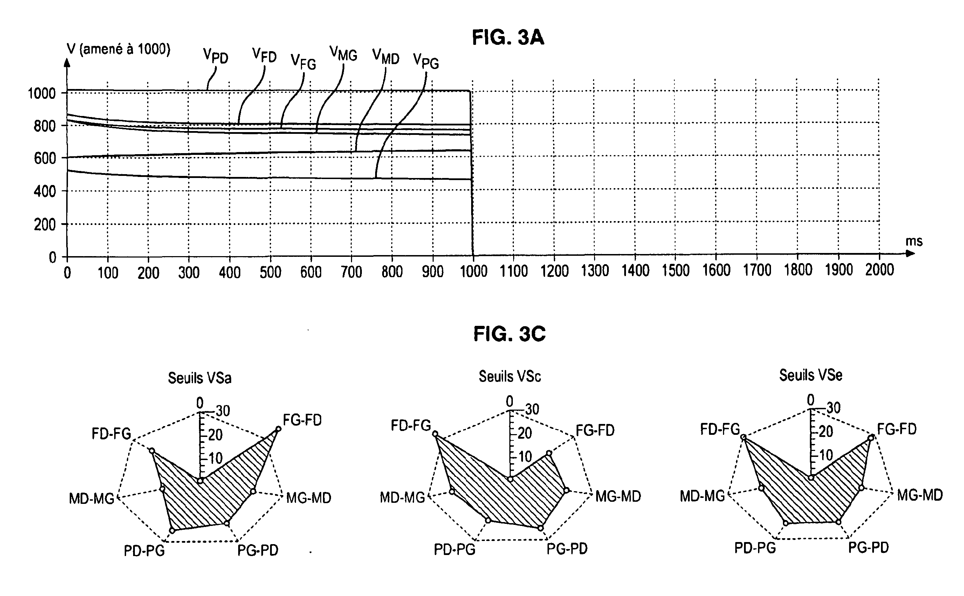

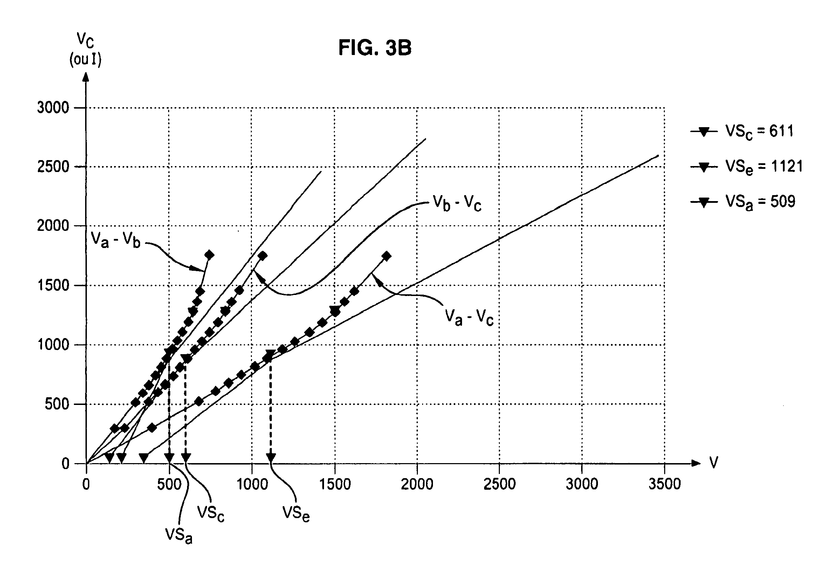

Ainsi les figures 3A et 3B des dessins illustrent des images écrans délivrées par un système de traitement de données (soit basé sur l'unité centrale 10, soit déporté) sur la base des données collectées.

La figure 3A illustre l'évolution des niveaux de tension relevés sur chacune des six électrodes, tensions désignées respectivement par V_{FG}, V_{FD}, F_{MG}, V_{MD}, V_{PG}, V_{PD}, pendant l'application d'un créneau (ici d'une durée d'une seconde) dans l'exemple où l'anode et la cathode Ea et Ec sont respectivement les électrodes PD et PG. On observe sur ces courbes que la tension V_{PD}, qui est la tension d'anode engendrée par le générateur 21, est bien constante sur toute la durée du créneau, tandis que les autres tensions mesurées varient initialement assez brutalement (en augmentant ou en diminuant), pour commencer à se stabiliser et être finalement stables à la fin du créneau. Ce sont les valeurs des tensions en fin de créneau, pour une pluralité de créneaux de niveaux de tension différents, qui sont utilisées pour établir les courbes illustrées sur la figure 3B.

Ces courbes illustrent respectivement l'évolution mutuelle de la tension de cathode (c'est-à-dire du courant I), figurant en ordonnées, et des différences de potentiel respectivement Va-Vb, Vb-Vc et Va-Vc, la tension figurant en abscisse. Ces courbes sont construites par le système de traitement de données en fonction des valeurs de tension obtenues en fin de chaque créneau de la série de créneaux appliquée comme décrit plus haut.

On observe que pour chacune de ces courbes, il existe une valeur seuil de tension (respectivement VSa, VSc et VSe) pour laquelle cette courbe quitte son allure linéaire pour s'incurver, ce qui est révélateur de l'apparition (ou de la disparition, dans le sens des tensions décroissantes) d'un phénomène électrochimique à l'anode ou à la cathode.

A partir de ces valeurs, qui correspondent chacune à une valeur de courant I donnée et donc à une valeur de la tension Vc donnée, il est possible d'obtenir le triplet de valeurs VSa, VSc et VSe correspondant à ces seuils.

Ces traitements étant effectués pour chacune des six paires d'électrodes choisies, on obtient pour un patient donné trois sextuplets de valeurs VSa, VSc et VSe correspondant à ces seuils.

En référence maintenant à la figure 3C, il est possible à partir de chacun de ces trois sextuplets de bâtir des graphiques tels que des courbes radar, qui permettent de facilement comparer visuellement les caractéristiques obtenues pour un patient donné avec des courbes modèles ou de référence, correspondant à des patients sains ou à des pathologies ou dispositions pathologiques données. Elles facilitent également la comparaison avec des caractéristiques obtenues précédemment avec le même patient, notamment pour le suivi de l'effet d'un traitement.

Bien entendu, l'homme du métier pourra imaginer de nombreux autres traitements sur les données collectées créneau par créneau comme décrit plus haut, voire entre les créneaux, sans sortir du cadre de la présente invention qui trouve sa spécificité dans la façon d'acquérir les données.

En outre, et comme on le décrira en détail dans la suite, les différents types de mesures peuvent être combinés de manières très diverses dans le cadre d'un procédé de traitement de données en vue de diagnostiquer telle ou telle pathologie, évolution pathologique ou prédisposition pathologique.

On va maintenant décrire une fonctionnalité de normalisation optionnellement prévue dans le système de l'invention, qui permet de s'affranchir de certains décalages de mesures de tensions (offset en terminologie anglo-saxonne) dus notamment à l'utilisation de matériaux d'électrodes différents ou à des matériaux dont le vieillissement a un impact sur le comportement au contact de la peau. On a observé en effet que selon la nature des matériaux d'électrode et leur degré d'usure ou de vieillissement, les interfaces électrode/peau constituaient des cellules électrochimiques engendrant des tensions variables (en plus ou en moins) de quelques dizaines à quelques centaines de millivolts, susceptibles de fausser les mesures. Cette fonctionnalité consiste, une fois que les électrodes ont été placées sur le patient, à relier l'une des électrodes à la source de tension, délivrant par exemple une valeur continue de 2 volts, à connecter les cinq autres électrodes en haute impédance, et à mesurer à l'aide du circuit de mesure les potentiels statiques sur ces cinq autres électrodes. Ces opérations sont répétées en appliquant la tension continue successivement à chacune des cinq autres électrodes.

On réalise de la sorte, par une opération rapide puisqu'elle nécessite quelques secondes par configuration d'électrodes, une cartographie qui permet de calculer des tensions de décalage qui seront utilisées pour corriger les mesures effectuées lorsque les créneaux de tensions seront appliqués au cours du processus d'acquisition décrit plus haut. Plus précisément, à chaque fois qu'une électrode donnée sera sélectionnée comme anode, les tensions de décalage relevées lorsque cette même électrode était reliée à la source de tension sont utilisées pour corriger les tensions relevées aux autres électrodes par le circuit de mesure.

De manière à minimiser la nécessité d'effectuer des corrections précitées, toutes les électrodes sont préférentiellement réalisées avec le même matériau. On choisit de préférence un matériau à forte teneur en nickel, dont on a pu déterminer par expériences in vitro qu'il était le plus approprié pour effectuer des mesures électrochimiques mettant en jeu les chlorures. Ceci s'explique probablement par un phénomène d'activation de la surface du substrat de nickel par les ions chlorures. Alternativement, on peut utiliser des électrodes en argent ou alliage riche en argent, donnant lieu à des couples électrochimiques (Ag/AgCl) qui peuvent contribuer à donner des mesures intéressantes.

La surface des électrodes est choisie aussi grande que pratiquement possible, sachant que les électrodes sont relativement rigides et doivent pouvoir être au contact de la peau sur toute leur étendue. Une surface de 2 à 100 cm² sera généralement choisie, en fonction de l'endroit du corps où l'électrode est destinée à être placée.

Bien entendu, de nombreuses variantes du système sont possibles.

On peut jouer notamment sur le nombre d'électrodes, et recourir par exemple à huit électrodes (quatre pour les extrémités des membres, deux pour le front et deux pour la poitrine).

En outre, l'architecture du système de l'invention peut être une architecture sans fil du type illustré sur la figure 3 du document FR-A-2 887 427 auquel on se réfèrera pour plus de détails.

De même, on peut associer à la présente invention un système de traitement temps réel ou quasi-réel local ou distant selon les principes décrits notamment dans le document précité.

On va maintenant décrire un exemple de procédé de traitement de donnée à des fins de diagnostic exploitant les mesures de phénomènes électrochimiques réalisées notamment avec un appareil tel que décrit ci-dessus, et pouvant être mis en oeuvre de façon locale ou distante.

De manière générale, le procédé consiste tout d'abord à acquérir des jeux de données multidimensionnelles mesurés sur une population de patients dont la ou les pathologies sont connues, et le cas échéant sur des patients déjà identifiés comme sains, à l'aide d'un système notamment tel que décrit plus haut. Ces mesures multidimensionnelles sont complétées avec des valeurs physiologiques, comportementales et/ou environnementales des patients telles que l'âge, le sexe, le poids, la consommation de tabac, la profession, le cadre de vie, etc.

Il est également possible de prendre en compte l'évolution de ces valeurs dans le temps, avec un espacement adapté (de quelques heures à plusieurs années).

Le procédé peut également prendre en compte des jeux de données multidimensionnelles avant et après un effort prédéterminé (test d'effort).

On crée ainsi un ensemble de jeux de données de référence pour un certain nombre de pathologies, par des techniques connues elles-mêmes d'extraction de données significatives (« data mining » en terminologie anglo-saxonnes).

Ces données comprennent typiquement des gammes de valeurs de référence selon les différentes dimensions, pour les différentes pathologies.

Avantageusement, ces jeux de données de référence peuvent être progressivement enrichis et/ou affinés à partir de nouvelles acquisitions sur patients atteints, par itération du processus d'extraction de données significatives.

En outre, à l'aide des techniques connues d'apprentissage, les jeux de données de références peuvent être affinés au fil du temps, par exemple sur la base d'outils de l'état de l'art tels que les arbres de décision, les réseaux de neurones ou les machines à vecteurs de support.

Chaque jeu de données multidimensionnelles obtenues avec le système décrit plus haut sur des patients à diagnostiquer, ou encore dont un diagnostic précédent demande une confirmation ou un suivi, et complété par les données additionnelles disponibles (saisies ou mémorisées par ailleurs) telles que le sexe et l'âge du patient, ses habitudes et son cadre de vie (et plus généralement toutes autres données physiologiques, comportementales, environnementales, etc.) peut alors être confronté aux jeux de données de référence mémorisés en association avec l'unité de traitement de données en charge de la mise en oeuvre du procédé, par toute technique de comparaison appropriée (somme pondérée ou non de scores de recouvrement ou de proximité avec les données de référence, etc.).

Préalablement à cette confrontation, et à nouveau de façon classique en soi, les données d'entrée sont si nécessaire normalisées.

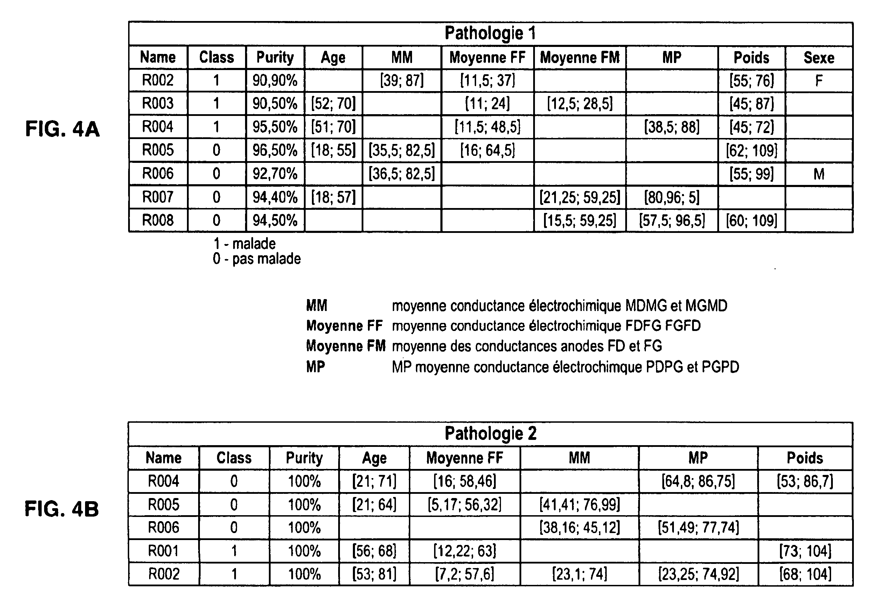

On a illustré sur le tableau de la figure 4A un jeu de données de référence obtenu pour une pathologie d'insuffisance rénale chronique.

Dans cet exemple et dans le suivant, ce jeu de données comprend un ensemble de N multiplets de gammes de valeurs significatives obtenues avec une série de patients atteints (Class = 1) et avec une série de patients non atteints (Class = 0), ces multiplets étant désignés par leurs noms R002 à R008 et formant un nuage de volumes multidimensionnels dans l'espace à N dimensions correspondant.

Dans cet exemple, les différentes dimensions sont les suivantes :
- l'âge du patient (AGE),
- MM : la moyenne des conductances électrochimiques MD-MG et MG-MD,
- FF : la moyenne des conductances électrochimiques FD-FG et FG-FD,
- FM : la moyenne des conductances des anodes FD et FG,
- MP : la moyenne des conductances électrochimiques PD-PG et PG-PD,
- le poids du patient (POIDS),
- le sexe du patient (SEX).

On notera ici que l'indice de pureté des données (« Purity » dans le tableau de la figure 4A) indique une probabilité que la règle utilisée corresponde à la classe indiquée...

Un autre exemple de jeu de données de référence relatif à une pathologie de type neuropathie autonome est illustré sur la figure 4B.

Dans un mode de réalisation particulier, le traitement de données en vue du diagnostic est effectué au niveau d'un serveur distant relié au système d'acquisition tel que décrit plus haut par un canal de communication sans fil (réseau local ou étendu tel que l'Internet ou GSM, avec sécurisation appropriée des données).

La restitution des informations de diagnostic peut être ensuite effectuée sur tout support, papier ou écran.

## Revendications

1. Système d'analyse électrophysiologique notamment pour la détection de pathologies, comprenant:
une série d'électrodes (E1-E6) destinées à être placées en différentes régions éloignées du corps humain,
une source de tension continue ajustable (21) commandée pour engendrer des créneaux successifs d'une tension continue qui varie d'un créneau à l'autre, la durée des créneaux étant supérieure ou égale à environ 0,2 seconde,
un circuit de commutation (30) agencé pour sélectivement relier une paire d'électrodes dites actives à la source de tension, et pour connecter au moins une autre électrode en haute impédance, et
un circuit de mesure (22, 40) agencé pour relever des données représentatives du courant dans les électrodes actives et des potentiels sur au moins certaines électrodes connectées en haute impédance en réponse à l'application desdits créneaux, et
un dispositif de traitement (10) agencé pour analyser l'évolution réciproque dudit courant et desdits potentiels en fonction de la tension des créneaux, et à comparer une telle évolution avec au moins une évolution de référence,
et en ce que la gamme des tensions couvertes par les créneaux est propre à provoquer d'un créneau à l'autre l'apparition ou la disparition de phénomènes électrochimiques au voisinage des électrodes actives.

2. Système selon la revendication 1, **caractérisé en ce que** le circuit de commutation (30) est agencé pour successivement relier différentes paires d'électrodes actives à ladite source de tension (21) et pour, lorsqu'une paire d'électrodes est reliée à la source de tension (21), connecter toutes les autres électrodes en haute impédance.

3. Système selon l'une des revendications 1 ou 2, **caractérisé en ce que** le circuit de mesure (22, 40) comprend une résistance (Rmes) agencée pour être connectée entre l'une des électrodes d'une paire active et une tension de référence et **en ce que** le système comprend également un circuit de calibration (40, 10) agencé, pour une paire d'électrodes actives données, pour ajuster la valeur de la résistance de mesure pour qu'elle soit du même ordre de grandeur que la résistance présente entre les deux électrodes actives en présence d'une tension continue, les données représentatives du courant dans les électrodes actives étant engendrées à partir de la différence de potentiel mesurée aux bornes de la résistance de mesure (Rmes).

4. Système selon la revendication 3, **caractérisé en ce que** le circuit de mesure (22, 40) est agencé pour mesurer les potentiels sur toutes les électrodes.

5. Système selon la revendication 4, **caractérisé en ce qu'**il comprend un circuit (10) de compensation de tensions de décalage agencé pour corriger les tensions mesurées à l'aide de tensions de correction obtenues en reliant individuellement chaque électrode à la source de tension, délivrant une tension continue constante, tandis que les autres électrodes sont connectées en haute impédance, et en mesurant les potentiels sur lesdites autres électrodes.

6. Système selon l'une des revendications 1 à 5, **caractérisé en ce que** les créneaux de tension ont une valeur de tension comprise entre environ 1 et 4 volts et une durée comprise entre environ 0,2 et 5 secondes.

7. Système selon la revendication 6, **caractérisé en ce qu'**il est agencé pour faire varier la tension des créneaux successifs dans un sens puis dans un autre et **en ce que** le système est agencé pour faire varier la tension des créneaux successifs avec un premier pas puis avec un deuxième pas plus petit que le premier.

8. Système selon l'une des revendications 6 à 7, **caractérisé en ce que** la tension des créneaux successifs varie par pas compris entre environ 0,05 et 1 volt et **en ce que** les créneaux successifs sont espacés d'une durée comprise entre environ 0,5 et 5 secondes.

9. Système selon l'une des revendications 1 à 8, **caractérisé en ce que** le circuit de commutation (30) est agencé pour relier une même paire d'électrodes à la source de tension selon deux polarités inversées et **en ce que** le système comprend deux électrodes pour lobes frontaux gauche et droit, deux électrodes pour mains gauche et droite et deux électrodes pour pieds gauche et droit, le circuit de commutation (30) étant agencé pour relier à la source de tension (21) des paires d'électrodes constituées par l'électrode de front gauche et l'électrode de front droit, l'électrode de front droit et l'électrode de front gauche, l'électrode de main gauche et l'électrode de main droite, l'électrode de main droite et l'électrode de main gauche, l'électrode de pied gauche et l'électrode de pied droit, et l'électrode de pied droit et l'électrode de pied gauche et **en ce qu'**après avoir relié une certaine paire d'électrodes à la source de tension avec une certaine polarité, le circuit de commutation (30) est agencé pour 10 relier cette même paire d'électrodes à la source de tension (21) avec une polarité inversée seulement après qu'une autre paire d'électrodes, distante sur le corps, a été reliée à la source de tension.

10. Procédé d'utilisation du système selon l'une des revendications précédentes comprenant les étapes suivantes :
- recevoir un jeu de données comprenant des mesures révélatrices de phénomènes électrochimiques au voisinage d'électrodes appliquées sur la peau du patient en des endroits du corps prédéterminés, dans lequel les données mesurées sont obtenues à partir de valeurs de courant dans des électrodes actives et de valeurs de potentiels sur des électrodes à haute impédance en réponse à l'application de créneaux de tension entre électrodes actives dont le niveau de tension varie d'un créneau à l'autre pour provoquer l'apparition ou la disparition de phénomènes électrochimiques au voisinage des électrodes actives et dans lequel lesdites mesures sont fournies par un système selon l'une des revendications 1 à 9,
- accéder à au moins un jeu de données de référence mémorisé comprenant des mesures révélatrices de phénomènes électrochimiques obtenues dans les mêmes conditions sur des patients identifiés comme atteints ou non atteints de cette pathologie, et
- confronter ledit jeu de donnés reçu avec les jeux de données de référence.

11. Procédé selon la revendication 10, dans lequel les jeux de données comprennent en outre des données d'ordre physiologique et/ou comportemental et/ou environnemental.

12. Procédé selon la revendication 10, dans lequel les jeux de données comprennent des mesures réalisées sur un patient après un effort prédéterminé du patient.

13. Procédé selon la revendication 12, lequel est mis en oeuvre dans un équipement informatique distant du système et relié à lui par un canal de communication de données.

## Claims

1. Electrophysiological analysis system in particular for the detection of pathologies, including:
a series of electrodes (E1-E6) intended to be placed at various regions of the human body distant from one another,
an adjustable DC voltage source (21) which is controlled in order to produce successive waves of a DC voltage which varies from one wave to the other, the duration of the waves being greater than or equal to approximately 0.2 second,
a switching circuit (30) designed to selectively connect a pair of so-called active electrodes to the voltage source, and to connect at least one other high-impedance electrode, and
a measuring circuit (22, 40) designed to collect data representative of the current in the active electrodes and of the potentials on at least some electrodes connected in high impedance, in response to the application of said waves, and
a processing device (10) designed to analyze the reciprocal evolution of said current and said potentials based on the wave voltage, and to compare such evolution with at least one reference evolution,
and in that the range of voltages covered by the waves is suitable for causing, from one wave to the other, the appearance or disappearance of electrochemical phenomena in the vicinity of the active electrodes.

2. System according to claim 1, **characterized in that** the switching circuit (30) is designed so as to successively connect various pairs of active electrodes to said voltage source (21) and, when a pair of electrodes is connected to the voltage source (21), for connecting all of the other high-impedance electrodes.

3. System according to one of claims 1 or 2, **characterized in that** the measuring circuit (22, 40) includes a resistor (Rmes) designed to be connected between one of the electrodes of an active pair and a reference voltage, and **in that** the system includes a calibration circuit (40, 10) which, for a given pair of active electrodes, is designed to adjust the value of the measurement resistor so that it is of the same order of magnitude as the resistance present between the two active electrodes in the presence of a DC voltage, in which the data representative of the current in the active electrodes is produced from the potential difference measured at the terminals of the measurement resistor (Rmes).

4. System according to claim 3, **characterized in that** the measuring circuit (22, 40) is designed to measure the potentials on all of the electrodes.

5. System of claim 4, **characterized in that** it includes an offset voltage-compensating circuit (10), which is designed to correct the measured voltages by means of correction voltages obtained by individually connecting each electrode to the voltage source, delivering a constant DC voltage, whereas the other electrodes are connected in high impedance, and by measuring the potentials on said other electrodes.

6. System according to one of claims 1 to 5, **characterized in that** the voltage waves have a voltage value of between approximately 1 and 4 volts, and a duration of between approximately 0.2 and 5 seconds.

7. System according to claim 6, **characterized in that** it is designed to vary the voltage of the successive waves in one direction and then in the other, and **in that** the system is designed to vary the voltage of the successive waves by a first step and then by a second step, which is smaller than the first.

8. System according to one of claims 6 or 7, **characterized in that** the voltage of the successive waves varies by steps of between approximately 0.05 and 1 volt and **in that** the successive waves are spaced apart by a duration of between approximately 0.5 and 5 seconds.

9. System according to one of claims 1 to 8, **characterized in that** the switching circuit (30) is designed to connect a single pair of electrodes to the voltage source in two reversed polarities, and **in that** it includes two electrodes for left and right frontal lobes, two electrodes for left and right hands and two electrodes for left and right feet, the switching circuit (30) is designed to connect, to the voltage source (21), electrode pairs consisting of the left forehead electrode and the right forehead electrode, the right forehead electrode and the left forehead electrode, the left hand electrode and the right hand electrode, the right hand electrode and the left hand electrode, the left foot electrode and the right foot electrode and the right foot electrode and the left foot electrode, and **in that** after having connected a certain pair of electrodes to the voltage source with a certain polarity, the switching circuit (30) is designed to connect this same pair of electrodes to the voltage source (21) with a reversed polarity, only after another distant pair of electrodes on the body has been connected to the voltage source.

10. Method for use of the system according to one of the previous claims, including the following steps:
- receiving a set of data comprising measurements revealing electrochemical phenomena in the vicinity of the electrodes applied to the skin of the patient at predetermined locations on the body, in which the data measured is obtained on the basis of current values in active electrodes and potential values on high-impedance electrodes in response to the application of voltage waves between active electrodes, of which the voltage level varies from one wave to the other to cause the appearance or disappearance of electrochemical phenomena in the vicinity of the active electrodes and in which said measurements are provided by a system according to one of claims 1 to 9,
- accessing at least one set of stored reference data comprising measurements revealing electrochemical phenomena obtained under the same conditions on patients identified as having or as not having this pathology, and
- reconciling said set of data received with the sets of reference data.

11. Process according to claim 10, in which the data sets further include data of a physiological and/or behavioral and or environmental nature.

12. Process according to claim 10, wherein the data sets comprise measurements taken on a patient after a predetermined exertion by the patient.

13. Process according to claim 12, which is implemented in computer equipment remote from the system and connected thereto via a data communication channel.

## Patentansprüche

1. Elektrophysiologisches Analysesystem, insbesondere zur Erfassung pathologischer Erscheinungen, aufweisend:
eine Reihe von Elektroden (E1-E6), die eingerichtet sind, an unterschiedlichen voneinander entfernten Bereichen des menschlichen Körpers platziert zu werden,
eine einstellbare Gleichspannungsquelle (21), die gesteuert wird, um aufeinanderfolgende Wellen einer von einer Welle zur anderen Welle variierenden Gleichspannung zu erzeugen, wobei die Dauer der Wellen größer oder gleich ca. 0,2 Sekunden ist,
eine Umschaltschaltung (30), die eingerichtet ist, selektiv ein Paar von sogenannten aktiven Elektroden mit der Spannungsquelle zu verbinden und zumindest eine andere Hochimpedanzelektrode anzuschließen, und
eine Messschaltung (22, 40), die eingerichtet ist, repräsentative Daten des Stroms in den aktiven Elektroden und Potenziale an zumindest bestimmten bei hoher Impedanz verbundene Elektroden im Ansprechen auf die Aufbringung der Wellen zu sammeln, und
eine Verarbeitungseinrichtung (10), die eingerichtet ist, den reziproken Verlauf des Strom und der Potenziale in Abhängigkeit von der Spannung der Wellen zu analysieren und einen solchen Verlauf mit zumindest einem Referenzverlauf zu vergleichen,
wobei der Spannungsbereich, der durch die Wellen gedeckt ist, dazu in der Lage ist, von einer Welle zu einer anderen Welle das Auftreten oder den Wegfall eines elektrochemischen Phänomens in dem Bereich der aktiven Elektroden zu bewirken.

2. System gemäß Anspruch 1, **dadurch gekennzeichnet, dass** die Umschaltschaltung (30) eingerichtet ist, sukzessive unterschiedliche Paare von den aktiven Elektroden mit der Spannungsquelle (21) zu verbinden und, wenn ein Paar der Elektroden mit der Spannungsquelle (21) verbunden ist, sämtliche von den anderen Hochimpedanzelektroden anzuschließen.

3. System gemäß einem der Ansprüche 1 oder 2, **dadurch gekennzeichnet, dass** die Messschaltung (22, 40) einen Widerstand (Rmes) aufweist, der eingerichtet ist, zwischen eine der Elektroden eines aktiven Paars und eine Referenzspannung geschaltet zu werden, wobei das System ebenfalls eine Kalibrierungsschaltung (40, 10) aufweist, die eingerichtet ist, für ein Paar aus gegebenen aktiven Elektroden den Wert des Messwiderstands derart einzustellen, dass letzterer in der gleichen Größenordnung wie der Widerstand ist, der zwischen den zwei aktiven Elektroden bei Vorliegen eines Gleichstroms vorliegt, wobei die repräsentativen Daten des Stroms in den aktiven Elektroden aus dem Potenzialunterschied erzeugt wird, der an den Anschlüssen des Messwiderstands (Rmes) gemessen wird.

4. System gemäß Anspruch 3, **dadurch gekennzeichnet, dass** die Messschaltung (22, 40) eingerichtet ist, die Potenziale an sämtlichen Elektroden zu messen.

5. System gemäß Anspruch 4, **dadurch gekennzeichnet, dass** es eine Offset-Spannung-Kompensationsschaltung (10) aufweist, die eingerichtet ist, die gemessenen Spannungen mittels Korrekturspannungen zu korrigieren, die durch individuelles Verbinden jeder Elektrode mit der einen konstanten Gleichstrom liefernden Spannungsquelle, während die anderen Elektroden bei hoher Impedanz verbunden sind und die Potenziale der anderen Elektroden gemessen werden, erhalten werden.

6. System gemäß einem der Ansprüche 1 bis 5, **dadurch gekennzeichnet, dass** die Spannungswellen einen Spannungswert haben, der zwischen ca. 1 und 4 Volt liegt, und eine Dauer haben, die zwischen ca. 0,2 und 5 Sekunden liegt.

7. System gemäß Anspruch 6, **dadurch gekennzeichnet, dass** es eingerichtet ist, zu bewirken, dass die sukzessiven Spannungswellen in die eine Richtung und in die andere Richtung variieren, wobei das System eingerichtet ist, zu bewirken, dass die sukzessiven Spannungsweller um einen ersten Schritt und dann um einen zweiten Schritt, welcher kleiner als der erste Schritt ist, variieren.

8. System gemäß einem der Ansprüche 6 bis 7, **dadurch gekennzeichnet, dass** die sukzessiven Spannungswellen um Schritte variieren, die zwischen ca. 0,05 und 1 Volt liegen, und dass die sukzessiven Spannungswellen voneinander um eine Dauer beabstandet sind, die zwischen ca. 0,5 und 5 Sekunden liegt.

9. System gemäß einem der Ansprüche 1 bis 8, **dadurch gekennzeichnet, dass** die Umschaltschaltung (30) eingerichtet ist, ein einziges Paar von Elektroden mit der Spannungsquelle gemäß zwei umgekehrten Polaritäten zu verbinden, und dass das System zwei Elektroden für linke und rechte Frontallappen, zwei Elektroden für linke und rechte Hände und zwei Elektroden für linke und rechte Füße aufweist, wobei die Umschaltschaltung (30) eingerichtet ist, an die Spannungsquelle (21) Elektrodenpaare anzuschließen, welche umfassen: die linke Frontalelektrode und die rechte Frontalelektrode, die rechte Frontalelektrode und die linke Frontalelektrode, die Elektrode für die linke Hand und die Elektrode für die rechte Hand, die Elektrode für die rechte Hand und die Elektrode für die linke Hand, die Elektrode für den linken Fuß und die Elektrode für den rechten Fuß und die Elektrode für den rechten Fuß und die Elektrode für den linken Fuß, und dass die Umschaltschaltung (30) eingerichtet ist, nach Verbindung eines bestimmten Paares von Elektroden mit der Spannungsquelle mit einer bestimmten Polarität dieses einzige Paar von Elektroden mit der Spannungsquelle (21) mit einer umgekehrten Polarität lediglich dann zu verbinden, nachdem ein anderes entferntes Paar von Elektroden an dem Körper mit der Spannungsquelle verbunden wurde.

10. Verfahren zur Verwendung des Systems gemäß einem der vorangehenden Ansprüche, wobei das Verfahren die folgenden Schritte aufweist:
- Empfangen von einem Datensatz, der Messungen aufweist, die elektrochemische Phänomene in der Umgebung der Elektroden bezeichnen, die an der Haut des Patienten an vorbestimmten Stellen des Körpers angebracht sind, wobei die gemessenen Daten auf der Grundlage von Stromwerten in den aktiven Elektroden und von Potenzialwerten an den Hochimpedanzelektroden im Ansprechen auf die Anlegung der Spannungswellen zwischen den aktiven Elektroden erhalten werden, deren Spannungsniveau von der einen Welle zu der anderen Welle variiert, um das Auftreten oder den Wegfall von elektrochemischen Phänomenen im Bereich der aktiven Elektroden zu bewirken, wobei die Messungen von einem System gemäß einem der Ansprüche 1 bis 9 geliefert werden,
- Zugreifen auf zumindest einen Satz von gespeicherten Referenzdaten, die Messungen umfassen, welche elektrochemische Phänomene bezeichnen, und welche unter den gleichen Bedingungen an den Patienten erhalten werden, die als an dieser pathologischem Erscheinung leidend oder nicht leidend erachtet werden, und
- Gegenüberstellen des empfangenen Satzes von Daten und der Sätze von Referenzdaten.

11. Verfahren gemäß Anspruch 10, bei dem die Datensätze weiterhin physiologische Daten und/oder Verhaltensdaten und/oder Umweltdaten umfassen.

12. Verfahren gemäß Anspruch 10, bei dem die Datensätze Messungen umfassen, die an einem Patient nach einer vorbestimmten Beanspruchung des Patienten vorgenommen wurden.

13. Verfahren gemäß Anspruch 12, welches in einer Computereinrichtung durchgeführt wird, die von dem System entfernt ist und die mit letzterem über einen Datenkommunikationskanal verbunden ist.
